# FASCICULE DE BREVET EUROPEEN

(11) **EP 2 068 724 B1**
(45) Date de publication et mention de la délivrance du brevet: **23.11.2016**
(21) Numéro de dépôt: 07848311.2
(22) Date de dépôt: 02.10.2007
(51) Int. Cl.: A61F 2/30, A61B 17/70, A61B 17/80

(54) **DISPOSITIF D'ALLONGEMENT INTRA-CORPOREL À AIMANT PERMANENT**
INTRAKORPORALE LÄNGLICHE VORRICHTUNG MIT PERMANENTMAGNET
INTRACORPOREAL ELONGATION DEVICE WITH A PERMANENT MAGNET

(30) Priorité: 03.10.2006 FR 0608678
(43) Date de publication de la demande: 17.06.2009
(73) Titulaire: Soubeiran, Arnaud, 75014 Paris (FR)
(72) Inventeur: Soubeiran, Arnaud, 75014 Paris (FR)
(74) Mandataire: Demulsant, Xavier
(86) Numéro de dépôt international: PCT/FR2007/001612
(87) Numéro de publication internationale: WO 2008/040880

(56) Documents cités:
- WO-A-01/78614
- WO-A-99/51160
- WO-A-2004/019796
- DE-U1- 8 515 687
- US-A- 3 976 060
- US-A- 6 033 412
- US-A1- 2005 261 779

## Description

### Domaine Technique

La présente invention se rapporte aux dispositifs d'allongement intra-corporels tels notamment les prothèses osseuses à croissances, les clous médullaires d'allongement osseux, les tiges rachidiennes de distraction ou de compression ou les distracteurs osseux ou inter-costaux et, plus particulièrement, à ceux qui comprennent un aimant permanent mobile pour la réception de l'énergie nécessaire à l'allongement par l'intermédiaire d'une source de champ magnétique placée à l'extérieur de l'organisme et agissant à travers la partie de l'organisme qui entoure ledit dispositif.

### Technique Antérieure

Plusieurs dispositifs d'allongement intra-corporels comprenant un aimant permanent mobile pour la réception de l'énergie nécessaire à l'allongement ont été proposés.

Pour la plupart, tels que ceux décrits dans les documents US 3 976 060, Proc Inst Mech Eng [H]. 1989;203(2):97-102, US 6 849 076, US 5 704 939 et FR 06/05236, ils sont constitués d'une première partie qui comprend des premiers moyens de liaison à l'organisme, d'une seconde partie qui comprend des seconds moyens de liaison à l'organisme et montée coulissante par rapport à ladite première partie, d'un aimant permanent, de moyens de liaison entre ledit aimant permanent et ladite première partie qui laisse audit aimant permanent un degré de liberté en rotation autour d'un axe sensiblement perpendiculaire à sa direction d'aimantation et de moyens pour transformer les mouvements dudit aimant permanent en mouvements de ladite seconde partie par rapport à ladite première partie.

Dans le document US 3 976 060 lesdits moyens de liaison entre ledit aimant permanent et ladite première partie du dispositif décrit sont un levier qui reçoit ledit aimant permanent à l'extrémité de son bras le plus long et l'axe autour duquel tourne ce levier et qui est perpendiculaire à la direction d'aimantation dudit aimant mais n'est pas un de ses axes principaux d'inertie. L'attraction-répulsion dudit aimant permanent au moyen d'une source de champ placée en dehors de l'organisme et qui présente successivement un pôle et l'autre sensiblement dans la direction d'aimantation dudit aimant permanent entraîne l'oscillation dudit levier. Si ladite direction d'aimantation coïncide avec la direction dans laquelle la distance entre ledit aimant permanent et l'extérieur de l'organisme est la plus courte la commande dudit dispositif est considérablement facilitée. Les moyens pour transformer les mouvements dudit aimant permanent en mouvements de ladite seconde partie par rapport à ladite première partie sont constitués d'un cliquet solidaire du bras le plus court opposé audit bras le plus long dudit levier, d'une roue dentée entraînée par ledit cliquet et d'une vis solidaire de ladite roue dentée et en liaison hélicoïdale avec ladite seconde partie et dont la rotation déboîte donc ladite seconde partie par rapport à ladite première partie. L'utilisation d'un levier comme moyen de réduction est intéressante mais ce dispositif doit rester étanche, particulièrement le volume balayé par l'aimant et le levier, mais aussi le système de cliquet, ce qui reste délicat à réaliser dans la durée et limite les modes de stérilisation possibles. En outre sa géométrie générale et son manque de compacité limitent sa résistance mécanique et ses possibilités d'implantation.

Dans les autres documents Proc Inst Mech Eng [H]. 1989;203(2):97-102, US 6 849 076, US 5 704 939 et FR 06/05236, ledit aimant permanent tourne autour d'un de ses axes principaux d'inertie qui est matérialisé sous la forme d'une pièce qui comporte un logement pour ledit aimant permanent et qui constitue lesdits moyens de liaison entre ledit aimant permanent et ladite première partie. L'allongement de ces dispositifs nécessite l'entraînement dudit aimant permanent sur 360°. Pour ce faire l'utilisation d'une source externe de champ magnétique qui entoure ou tourne autour de toute la partie de l'organisme contenant ledit dispositif est nécessaire. Ladite source doit être dimensionnée en fonction de la plus grande dimension diamétrale relativement à l'axe de rotation dudit aimant permanent de ladite partie de l'organisme qui contient ledit dispositif ce qui n'est pas favorable à l'encombrement, à la puissance, au coût, à l'optimisation pour un patient donné ou à l'ergonomie de ladite source. Tout cela sera particulièrement sensible si le dispositif est, par exemple, placé le long de la colonne vertébrale: entourer le thorax ou tourner autour est bien moins avantageux que de pouvoir activer le dispositif en plaçant la source au plus près dans le dos.

Dans les documents US 5 704 939 et FR 06/05236, les moyens pour transformer les mouvements dudit aimant permanent en mouvements de ladite seconde partie par rapport à ladite première partie des dispositifs décrits sont une simple vis solidaire desdits moyens de liaison entre ledit aimant permanent et ladite première partie et d'axe confondu avec celui de la rotation dudit aimant permanent. Ces dispositifs peuvent fonctionner sans joint et restent simples mais la force de distraction produite est limitée.

Dans les documents Proc Inst Mech Eng [H]. 1989;203(2):97-102 et US 6 849 076, les moyens pour transformer les mouvements dudit aimant permanent en mouvements de ladite seconde partie par rapport à ladite première partie décrits sont constitués, outre d'une vis comme dans les documents US 5 704 939 et FR 06/05236, d'un réducteur à engrenage intercalé entre ladite vis et lesdits moyens de liaison entre ledit aimant permanent et ladite première partie. La force de distraction que ces dispositifs peuvent produire est élevée mais ledit réducteur est coûteux, nécessite des moyens de production spécifiques, est encombrant et doit rester étanche pour un bon fonctionnement. La miniaturisation de ces dispositifs est en outre très délicate.

Le document DE 6 85 15 687 U1 décrit les caractéristiques du préambule des revendications indépendantes ci-après.

Les objectifs du dispositif d'allongement intra-corporel selon l'invention sont donc:
- un fonctionnement sans joint, même envahi par les matières biologiques produites par son environnement, car assurer l'étanchéité d'un dispositif d'allongement intra-corporel reste une difficulté et une faiblesse et restreint les possibilités de stérilisation.
- une force de distraction élevée dans un volume modeste pour permettre l'utilisation dans tous les types d'allongements, notamment les allongements osseux, et toutes les localisations, y compris en pédiatrie. L'effort d'allongement produit par un dispositif d'allongement à transmission magnétique dépendant, d'une part, de l'effort exercé sur ledit aimant permanent par ladite source de champ magnétique placée à l'extérieur de l'organisme et, d'autre part, des moyens de réduction utilisés et l'effort exercé sur ledit aimant permanent dépendant de la puissance de la source externe de champ magnétique mais également, fortement, de la distance à laquelle elle peut être placée par rapport audit aimant permanent, le dispositif selon l'invention combinera des moyens de réduction simples, compacts et puissants avec la possibilité de commander ledit dispositif depuis la direction perpendiculaire à l'axe de rotation dudit aimant dans laquelle ledit aimant permanent est le plus proche de l'extérieur de l'organisme.
- une bonne ergonomie, tant pour faciliter son implantation par le chirurgien que son allongement le plus souvent à domicile, ou même itinérant, par le patient lui-même, notamment dans les traitements, tels les allongements osseux, qui nécessitent un allongement au moins quotidien, avec, en particulier, des moyens externes pour commander l'allongement et une mise en oeuvre des dits moyens surs et adaptés à un tel usage quotidien et non qualifié.
- un coût raisonnable, ce qui est notamment lié au nombre et à la difficulté de réalisation des pièces du dispositif. Les systèmes à cliquet, à engrenage ou à joint qui nécessitent des techniques de production particulières et une qualité de surface particulière, naturellement plus coûteux que ceux qui comportent un nombre limité de pièces réalisables avec des techniques de production courantes, d'autant plus que le système doit être de petite taille, seront évités. Les composants du dispositif selon l'invention devront donc être réalisables dans un atelier de micro-mécanique courant et ceci quelle que soit l'échelle à laquelle il sera utile de produire ledit dispositif.

### Exposé de l'invention

Pour ce faire, le dispositif d'allongement intra-corporel suivant l'invention, est défini dans les revendications indépendantes ci-après.

Lesdits moyens de transmission peuvent être une première liaison hélicoïdale entre une première surface solidaire dudit arbre de sortie et une seconde surface solidaire de ladite seconde partie.

Lesdits moyens démultiplicateurs peuvent être constitués d'un levier solidaire dudit arbre d'entrée dont il partage l'axe de rotation et de moyens de liaison entre une première surface d'appui solidaire dudit aimant permanent et distante de son axe de rotation et une seconde surface d'appui solidaire de l'extrémité dudit levier la plus éloignée dudit axe de rotation commun audit levier et audit arbre d'entrée.

La rotation dudit aimant permanent peut être limitée à un secteur d'angle inférieur à 180° par des premiers moyens de butée dans un sens et des seconds moyens de butée dans le sens opposé.

Lesdits moyens de liaison entre ladite première surface d'appui et ladite seconde surface d'appui peuvent comporter un jeu qui permet la rotation dudit aimant permanent sans entraînement dudit levier sur deux secteurs sensiblement opposés l'un à l'autre par rapport à l'axe de rotation dudit aimant permanent.

Lesdits moyens démultiplicateurs peuvent alternativement être constitués d'une pièce amplificatrice montée en translation et bloquée en rotation par rapport à ladite première partie et comportant une troisième surface en seconde liaison hélicoïdale avec une quatrième surface solidaire dudit aimant permanent et une cinquième surface en troisième liaison hélicoïdale avec une sixième surface solidaire dudit arbre d'entrée, les axes de rotation dudit aimant permanent, des dites seconde et troisième liaisons hélicoïdales et dudit arbre d'entrée étant confondus, les pas des dites seconde et troisième liaisons hélicoïdales étant dans ledit rapport de réduction desdits moyens démultiplicateurs et le pas de ladite seconde liaison hélicoïdale étant suffisamment grand relativement au coefficient de friction de ladite seconde liaison hélicoïdale pour permettre le fonctionnement desdits moyens démultiplicateurs.

Quelle que soit sa forme de réalisation, le dispositif suivant l'invention pourra être commandé à l'aide d'une source de champ magnétique placée à l'extérieur de l'organisme et apte à produire un champ tournant, soit autour de l'axe de rotation dudit aimant permanent, soit autour d'un axe qui lui est parallèle. Ladite source de champ magnétique pourra être une source électromagnétique mais sera préférentiellement un aimant néodyme placé à l'extérieur de l'organisme au point le plus proche dudit aimant permanent et de manière à ce qu'un de ses axes principaux d'inertie sensiblement perpendiculaire à sa direction d'aimantation soit sensiblement parallèle à l'axe de rotation dudit aimant permanent. Ledit aimant néodyme sera alors mis en rotation autour de cet axe principal d'inertie parallèle à l'axe de rotation dudit aimant permanent. Alternativement ledit aimant néodyme pourra tourner tout autour de la partie de l'organisme contenant ledit dispositif suivant l'invention sensiblement suivant l'axe de rotation dudit aimant permanent et en lui présentant constamment le même pôle. Dans ce dernier cas, il pourra être avantageux qu'un deuxième aimant néodyme soit maintenu suffisamment distant du premier par une structure qui peut comporter un matériau magnétique doux, le pôle nord de l'un faisant face au pôle sud de l'autre pour qu'il puisse être disposés à l'extérieur de l'organisme, de part et d'autre dudit dispositif suivant l'invention.

Suivant la localisation du dispositif, la morphologie du patient et la forme de réalisation du dispositif suivant l'invention l'une ou l'autre des méthodes sera plus efficace. Si, par exemple, lesdits moyens démultipticateurs sont constitués d'une dite pièce amplificatrice alors ledit champ tournant devra tourner d'un certain nombre de tours dans une direction, puis du même nombre de tours dans la direction opposée pour commander ledit dispositif suivant l'invention, et ainsi de suite, et il sera préférable que cette rotation soit effectuée autour de l'axe de rotation dudit aimant permanent. Dans ce cas, on choisira donc préférentiellement de faire tourner ledit aimant néodyme tout autour de la partie d'organisme contenant ledit dispositif en présentant toujours un même pôle tourné vers l'axe de rotation dudit aimant permanent, sauf si ladite partie de l'organisme présente des zones très proches dudit aimant permanent et d'autres très éloignées. C'est le cas, par exemple, pour une tige rachidienne, pour laquelle on choisira préférentiellement, soit une autre forme de réalisation du dispositif, soit de commander le dispositif suivant la méthode consistant à faire tourner ledit aimant néodyme autour de son axe principal d'inertie parallèle audit axe de rotation dudit aimant permanent du nombre de tours nécessaires dans un sens puis dans l'autre et ainsi de suite. Si, lesdits moyens démultiplicateurs sont constitués d'un levier, la méthode consistant à faire tourner ledit aimant néodyme autour de son axe principal d'inertie parallèle audit axe de rotation dudit aimant permanent sera généralement préférée et le sens de rotation aura peu d'importance sauf si lesdits moyens de liaison entre ladite première surface d'appui et ladite seconde surface d'appui comportent un jeu, cas dans lequel un sens produira une force de distraction légèrement supérieure à l'autre sens.

Si la rotation dudit aimant permanent est limitée à un secteur d'angle inférieur à 180° par des premiers moyens de butée dans un sens et des seconds moyens de butée dans le sens opposé alors, le dispositif suivant l'invention peut être commandé par les deux méthodes déjà décrites mais aussi en présentant successivement chacun des deux pôles de ladite source de champ magnétique sensiblement dans la direction perpendiculaire à la direction bissectrice des directions dans lesquelles se trouvent ladite direction d'aimantation dudit aimant permanent quand celui-ci est respectivement, dans sa position correspondant aux dits premiers moyens de butée et, dans sa position correspondant aux dits seconds moyens de butée.

### Description sommaire des dessins

L'invention, son fonctionnement et ses applications seront mieux compris et d'autres de ses caractéristiques et avantages apparaîtront au cours de la description suivante donnée en regard des dessins annexés à titre illustratif mais nullement limitatif dans lesquels :

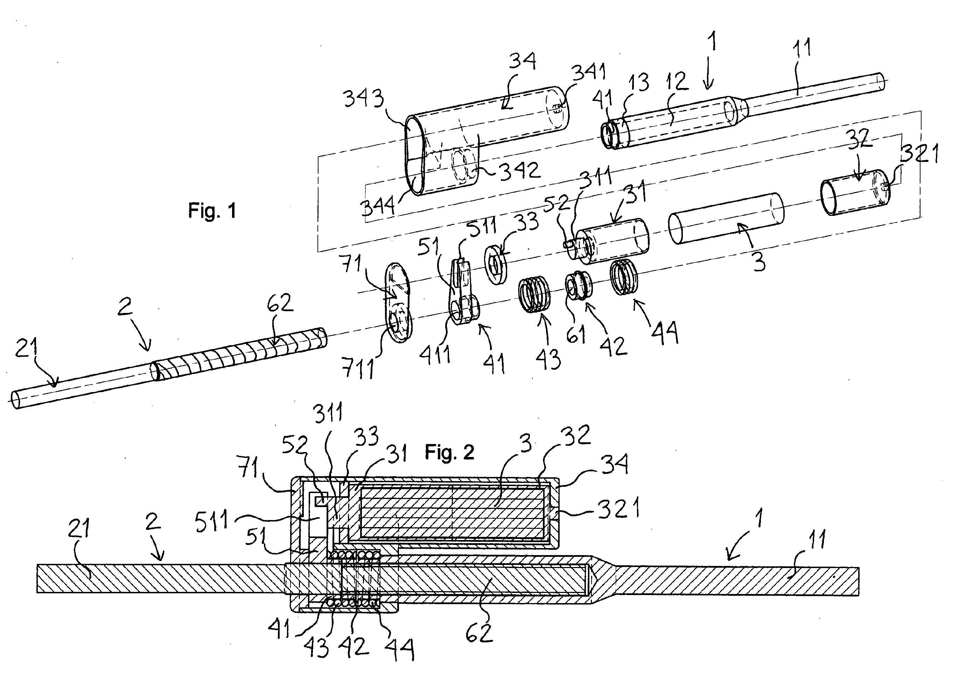

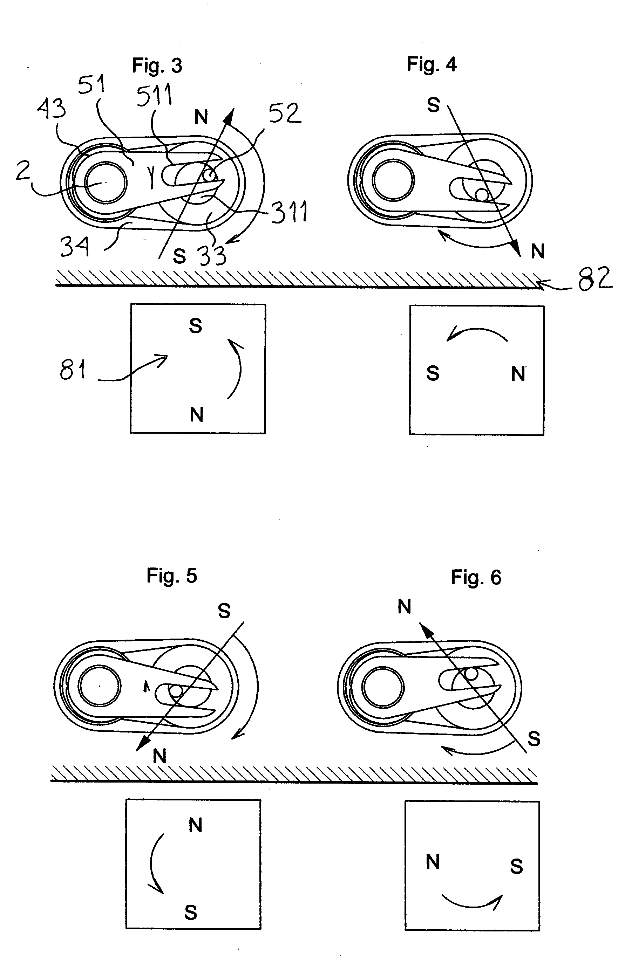

Les figures 1 à 6 représentent un premier mode de réalisation préféré du dispositif suivant l'invention plus particulièrement adapté à l'implantation à la surface des os, par exemple le long de la colonne vertébrale, de la mâchoire ou entre des côtes. La figure 1 est une vue éclatée en perspective de ce mode de réalisation. La figure 2 en est une vue en coupe passant par les axes de rotation dudit aimant permanent et de translation de ladite seconde partie par rapport à ladite première partie et dans lequel ledit dispositif est représenté dans sa position raccourcie. Les figures 3 à 6 sont des vues suivant l'axe de rotation dudit aimant permanent dans lesquelles le dispositif est présenté sans certains de ses composants pour laisser apparaître ledit levier dans quatre états différents en présence d'une source de champ magnétique placée à l'extérieur de l'organisme.

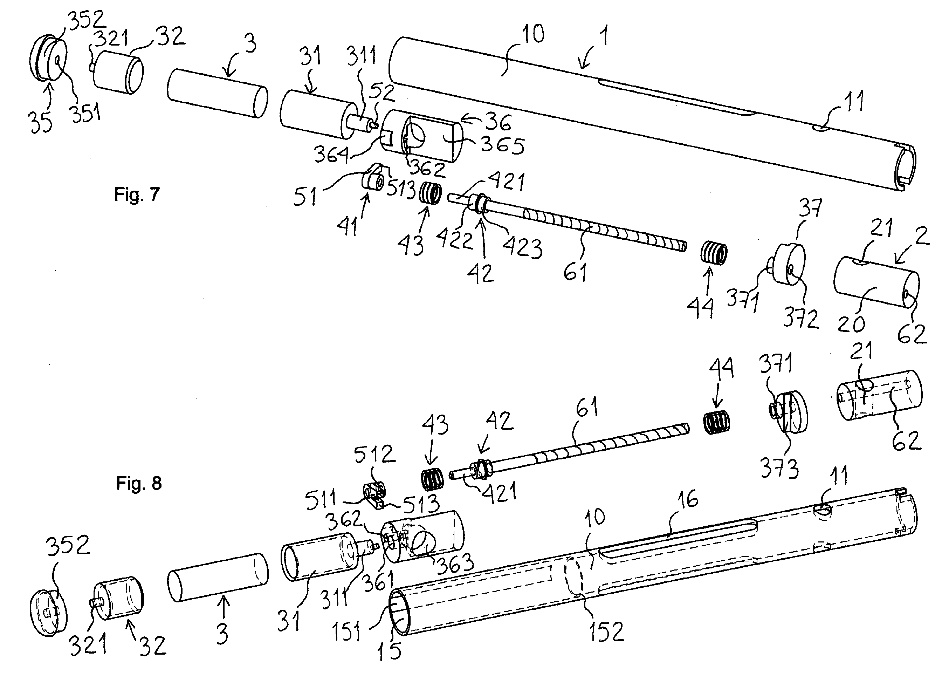

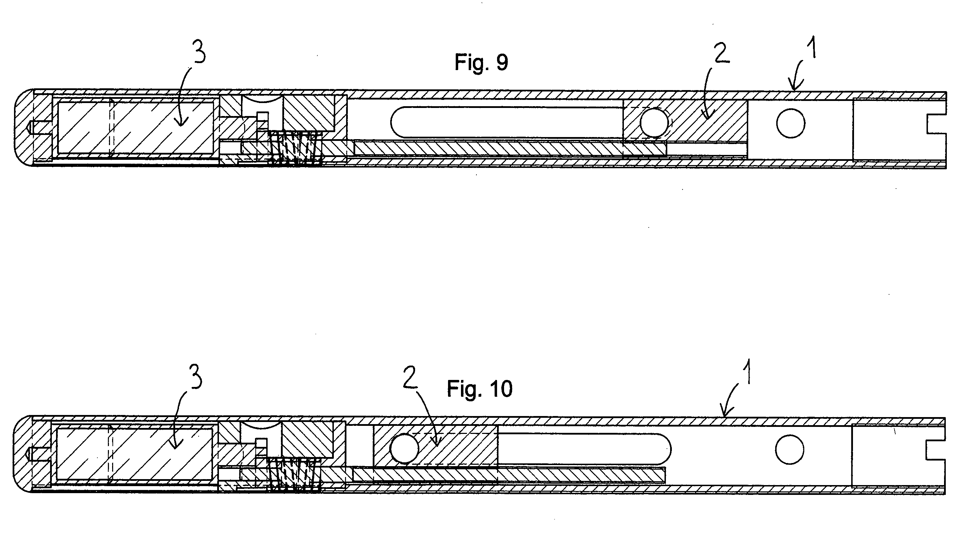

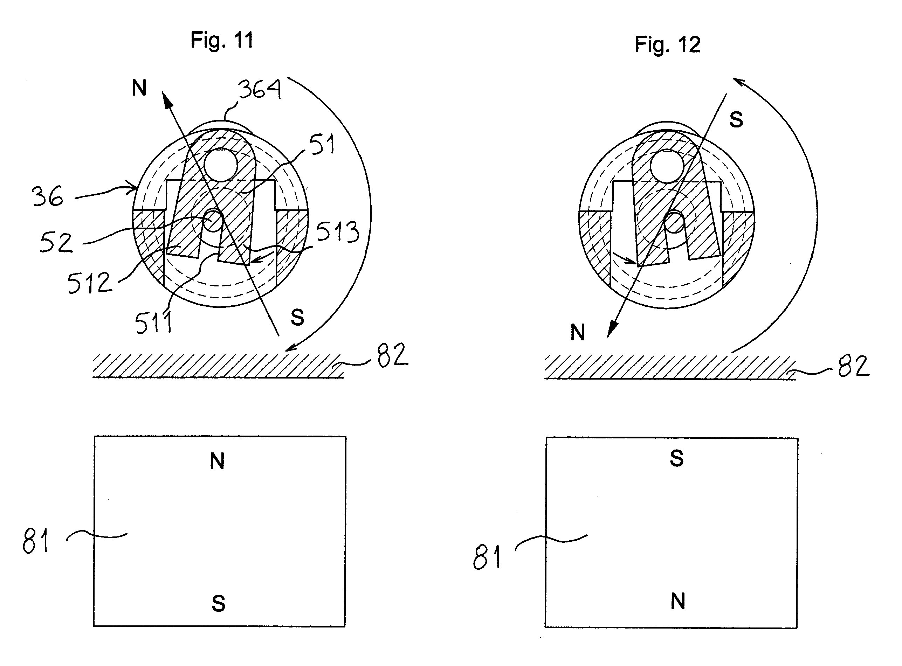

Les figures 7 à 12 représentent un second mode de réalisation préféré du dispositif suivant l'invention plus particulièrement adapté à l'implantation dans le canal médullaire d'un os long pour réaliser un allongement ou un transport osseux. Les figures 7 et 8 sont des vues éclatées en perspective de ce second mode de réalisation suivant deux angles différents. Les figures 9 et 10 en sont des vues en coupe passant par l'axe de rotation dudit aimant permanent. La figure 9 représente ledit second mode de réalisation préféré du dispositif suivant l'invention dans sa position initiale, la figure 10 le représente en fin d'allongement. Les figures 11 et 12 sont des coupes dudit second mode de réalisation préféré passant par ledit levier et montrant deux états dudit dispositif en présence d'une source de champ magnétique placée à l'extérieur de l'organisme.

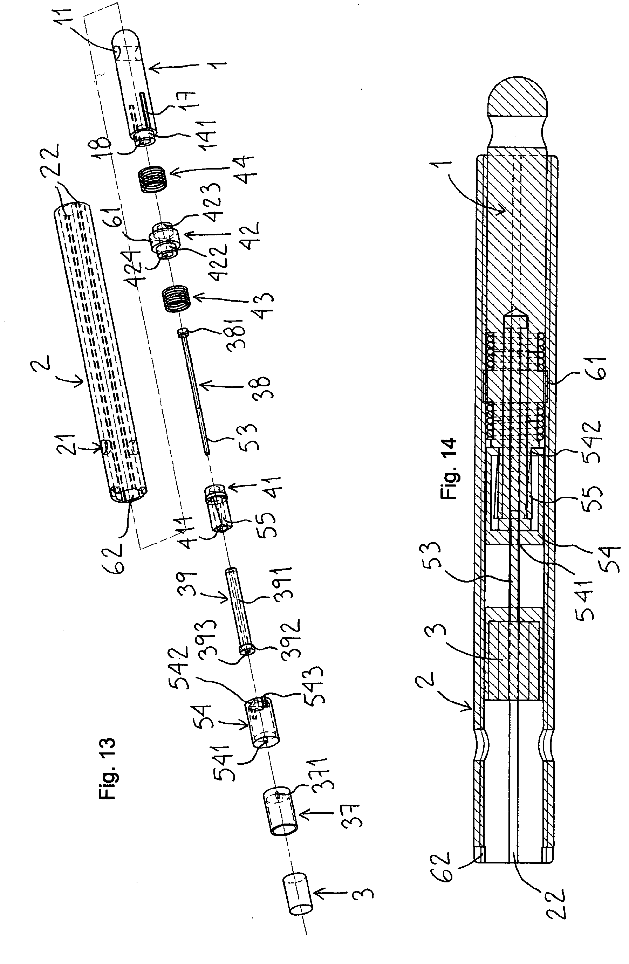

Les figures 13 et 14 représentent un troisième mode de réalisation préféré du dispositif suivant l'invention particulièrement utile quand l'importance de la force d'allongement produite prime sur l'ergonomie de commande. La figure 13 représente en perspective un éclaté dudit troisième mode de réalisation. La figure 14 représente une coupe dudit troisième mode de réalisation préféré passant par l'axe de rotation dudit aimant permanent.

Il est bien précisé que, sur les figures, les mêmes références désignent les mêmes éléments, quelle que soit la figure sur laquelle elles apparaissent et quelle que soit la forme de représentation de ces éléments. De même, si un élément n'est pas spécifiquement référencé sur l'une des figures pour ne pas surcharger cette dernière, sa référence peut être aisément retrouvée en se reportant à une autre figure.

Les lignes cachées sont représentées en pointillés dans toutes les figures sauf dans les figures 3 à 7 où elles ne sont pas représentées. Dans toutes les figures, les dimensions et proportions ont été adaptées quand cela pouvait faciliter la compréhension.

Le demandeur tient aussi à préciser que les figures représentent plusieurs modes de réalisation préférés du dispositif selon l'invention mais qu'il existe d'autres modes de réalisation qui répondent à la définition de cette invention.

Il précise en outre que, lorsque que, selon la définition de l'invention, le dispositif suivant l'invention comporte « au moins un » élément ayant une fonction donnée, le mode de réalisation décrit peut comporter plusieurs de ces éléments.

Il précise aussi que si les modes de réalisation de l'objet selon l'invention tels qu'illustrés comportent plusieurs éléments de fonctions identiques et que si, dans la description, il n'est pas spécifié que l'objet selon cette invention doit obligatoirement comporter un nombre particulier de ces éléments, l'objet de l'invention pourra être défini comme comportant « au moins un » de ces éléments.

### Manières de réaliser l'invention

Tous les modes de réalisation du dispositif selon l'invention sont constitués:
- d'une première partie **1** qui comprend des premiers moyens **11** de liaison à l'organisme,
- d'une seconde partie **2** qui comprend des seconds moyens **21** de liaison à l'organisme et montée coulissante par rapport à ladite première partie **1,**
- d'un aimant permanent **3**
- de moyens de liaison entre ledit aimant permanent **3** et ladite première partie 1 qui laissent audit aimant permanent **3** un degré de liberté en rotation sensiblement autour d'un de ses axes principaux d'inertie perpendiculaire à sa direction d'aimantation,
- un arbre d'entrée **41,**
- de moyens démultiplicateurs pour transformer les mouvements dudit aimant permanent 3 en rotation dudit arbre d'entrée **41** alternativement dans un premier sens et /ou dans le sens opposé avec un rapport de réduction entre la rotation dudit arbre d'entrée **41** et celle dudit aimant permanent **3** et, dans les Figures 13 et 14, tels qu'un changement de sens de la rotation dudit aimant permanent **3** entraîne le changement de sens de la rotation dudit arbre d'entrée **41,**
- un arbre de sortie **42,**
- des moyens de roue libre **43** aptes à entraîner ledit arbre de sortie **42** dans ledit premier sens quand ledit arbre d'entrée **41** tourne dans ledit premier sens,
- des moyens de maintien **44** pour empêcher la rotation dudit arbre de sortie **42** dans le sens opposé audit premier sens,
- et des moyens de transmission pour que la rotation dudit arbre de sortie **42** entraîne le déplacement de ladite seconde partie 2 par rapport à ladite première partie **1.**

En outre, dans toutes les formes de réalisation préférées représentées sur les figures 1 à 14 lesdits moyens de transmission sont une première liaison hélicoïdale entre une première surface **61** solidaire dudit arbre de sortie **42** et une seconde surface **62** solidaire de ladite seconde partie **2.**

Dans le premier mode de réalisation préféré du dispositif représenté sur les figures 1 à 6, ladite première partie **1** est constituée sensiblement d'un tube **12** prolongé à l'une de ses extrémités par une tige **11** qui constitue lesdits premiers moyens **11** de liaison à l'organisme. Une vis **62,** dont le diamètre extérieur correspond sensiblement au diamètre intérieur dudit tube **12** et prolongée d'une tige qui constitue lesdits seconds moyens **21** de liaison à l'organisme, constitue ladite seconde partie **2.**

L'aimant permanent **3** est cylindrique, aimanté diamétralement et collé, par exemple à l'aide d'une colle silicone, à l'intérieur de deux premier **31** et second **32** capuchons tête-bêches qui, à l'extrémité opposée à celle dans laquelle est inséré ledit aimant permanent **3,** sont respectivement prolongés, par un premier axe **311** qui matérialise l'axe de rotation dudit aimant permanent **3** puis par un axe excentrique **52,** pour ledit premier capuchon **31,** et par un second axe **321** qui matérialise l'axe de rotation dudit aimant permanent **3** seulement, pour ledit second capuchon **32.**

Un carter **34** comportant deux première **343** et seconde **344** cavités cylindriques parallèles, reçoit dans ladite première cavité **343** l'assemblage des deux premier **31** et second **32** capuchons et dudit aimant permanent **3,** ledit second axe **321** dudit second capuchon **32** en premier qui vient se loger dans un perçage **341** en fond de ladite première cavité cylindrique **343.** Une rondelle **33** dont le diamètre extérieur correspond au diamètre intérieur de ladite première cavité cylindrique **343** et le diamètre intérieur à celui dudit premier axe **311** vient se placer autour dudit premier axe **311** et fait office de palier.

Ladite seconde cavité cylindrique **344** dudit carter **34** reçoit à travers un perçage d'assemblage **342** situé du côté où est placé ledit second axe **321** dudit second capuchon **32** l'extrémité tubulaire de ladite première partie **1** dudit premier mode de réalisation préféré du dispositif suivant l'invention. Une surface cylindrique **13** de ladite première partie **1** de diamètre légèrement supérieur au diamètre dudit perçage d'assemblage **342** bloque ladite première partie **1** dans ledit carter de manière à ce que seul émerge dans ladite seconde cavité cylindrique **344** une surface cylindrique de friction **14** située à l'extrémité de ladite première partie **1.**

Les deux capuchons **31, 32,** le carter **34** et ladite rondelle **33** constituent ainsi les moyens de liaison entre ledit aimant permanent **3** et ladite première partie **1** qui laissent audit aimant permanent 3 un degré de liberté en rotation sensiblement autour d'un de ses axes principaux d'inertie perpendiculaire à sa direction d'aimantation.

Du côté opposé à celui dans lequel elle reçoit ladite première partie **1,** ladite seconde cavité cylindrique **344** dudit carter **34** reçoit, dans l'ordre et emboîtés les uns derrière les autres, un ressort de maintien **44,** un écrou constituant ledit arbre de sortie **42,** un ressort de roue libre d'entraînement **43** puis ledit arbre d'entrée **41** constitué d'un segment de tube dont l'intérieur **411** est apte à coulisser et tourner sur ladite vis **62** et dont la surface extérieure est apte à coopérer avec ledit ressort de roue libre d'entraînement **43** qui coopère aussi avec une surface analogue située sur ledit écrou **42** pour constituer lesdits moyens de roue libre **43** aptes à entraîner ledit arbre de sortie **42** dans ledit premier sens quand ledit arbre d'entrée **41** tourne dans ledit premier sens.

Ledit ressort de maintien **44** coopère avec ladite surface cylindrique de friction **14** située à l'extrémité de ladite première partie 1 et une surface analogue située sur ledit écrou **42** pour constituer lesdits moyens de maintien pour empêcher la rotation dudit arbre de sortie **42** dans le sens opposé audit premier sens.

Le détail et le fonctionnement de ce système à ressorts de friction est entièrement décrit dans le document WO/2004/019796 et ne sera en conséquence pas plus détaillé ici. Ce système répond bien aux objectifs fixés pour la présente invention mais peut, bien sur, aussi être remplacé par tout type de système à roue libre, par exemple des roues libres à aiguilles.

Ledit écrou **42** coopère, à travers son filetage intérieur **61,** avec la vis **62** constituant lesdits moyens de transmission pour que la rotation dudit arbre de sortie **42** entraîne le déplacement de ladite seconde partie **2** par rapport à ladite première partie **1.**

Un levier **51** solidaire dudit arbre d'entrée **41** dont il partage l'axe de rotation, constitué dans ce premier mode de réalisation préféré du dispositif par ladite vis **62,** comporte une gorge d'appui **511** dont la largeur est supérieure ou égale au diamètre dudit axe excentrique **52** avec lequel elle coopère pour constituer lesdits moyens démultiplicateurs pour transformer les mouvements dudit aimant permanent **3** en rotation dudit arbre d'entrée **41** alternativement dans un premier sens et dans le sens opposé avec un rapport de réduction entre la rotation dudit arbre d'entrée **41** et celle dudit aimant permanent **3** et tels qu'un changement de sens de la rotation dudit aimant permanent **3** entraîne le changement de sens de la rotation dudit arbre d'entrée **41,**

Une pièce d'obturation **71** comprenant un perçage **711** pour laisser passer ladite seconde partie 2 ferme le carter une fois le reste de l'assemblage achevé.

Le fonctionnement de ces dits moyens démultiplicateurs est détaillé sur les figures 3 à 6 où les flèches indiquent le mouvement qui va se produire sous l'effet de la position qui est représentée de la source de champ magnétique **81** placée à l'extérieur de l'organisme et dont on suppose qu'elle vient d'être donnée. Les limites **82** de l'organisme sont symbolisées sur lesdites figures par un trait gras représentant la peau et des hachures du coté de l'intérieur de l'organisme. La figure 3 représente le début d'un temps moteur ou ledit levier **51** va être entraîné par ledit axe excentrique **52** qui est arrivé au contact de la gorge d'appui **511** dans le sens des aiguilles d'une montre dans ledit premiers sens jusqu'à un maximum de déplacement dudit levier dans ce premier sens représenté sur la figure 4. Entre la figure 4 et la figure 5, l'aimant permanent poursuit sa rotation dans le sens des aiguilles d'une montre mais sans entraîner le levier **51,** du fait de la largeur plus importante que le diamètre dudit axe excentrique **52** de la gorge d'appui **511,** jusqu'à ce que ledit axe excentrique **52** revienne buter sur la face opposée à celle qu'il a quittée de la gorge d'appui **511** et qu'un nouveau temps moteur qui conduit à la position maximale du levier **51** dans le sens opposé audit premier sens représentée sur la figure 6 débute. Un nouveau secteur de rotation de l'aimant permanent **3** pendant lequel ledit levier **51** n'est pas entraîné suit et le système retrouve la position représentée sur la figure 3 avant d'entamer un nouveau cycle. La position relative des directions d'aimantation respectives dudit aimant permanent **3** et de de ladite source de champ magnétique **8** placée à l'extérieur de l'organisme conditionne l'intensité des efforts qui peuvent être transmis de l'un à l'autre. Si l'on veut pouvoir actionner efficacement ledit premier mode de réalisation préféré du dispositif suivant l'invention à l'aide d'un aimant néodyme placé à l'extérieur de l'organisme au point le plus proche dudit aimant permanent **3,** l'existence de secteurs dans lesquels la rotation de l'aimant permanent **3** n'entraîne pas le levier **51** permet de n'utiliser la rotation de l'aimant permanent **3** pour allonger ledit dispositif ou le réarmer que dans les secteurs où l'intensité du couple qui lui est appliqué est suffisante pour effectuer ces efforts moteurs.

On se réfère maintenant aux figures 7 à 12 qui représentent un second mode de réalisation préféré du dispositif suivant l'invention plus particulièrement adapté à l'implantation dans le canal médullaire d'un os long pour réaliser un allongement ou un transport osseux. Dans ce second mode de réalisation préféré, ladite première partie **1** est constituée sensiblement d'un tube **10** qui comporte à une première extrémité une cavité cylindrique **15** de diamètre plus important que le reste de l'intérieur dudit tube **10** et qui se termine par une surface annulaire d'appui **152** et au moins une gorge intérieure longitudinale **151** sensiblement de même longueur que ladite cavité cylindrique **15.**

Sensiblement à l'extrémité opposée à ladite première extrémité, au moins un perçage formant un angle compris entre 30 et 90° par rapport à l'axe dudit tube **10** constitue lesdits premiers moyens **11** de liaison à l'organisme. Ladite seconde partie **2** est sensiblement un cylindre **20** apte à coopérer avec le petit diamètre dudit tube **10.** Lesdits seconds moyens **21** de liaison à l'organisme sont constitués d'au moins un perçage sensiblement perpendiculaire à l'axe dudit cylindre **20.** Des découpes longitudinales oblongues **16** sont pratiquées dans ledit tube **10** pour permettre le verrouillage dans lesdits seconds moyens de liaison **21** et l'allongement ultérieur.

Comme dans ledit premier mode préféré de réalisation du dispositif suivant l'invention, l'aimant permanent **3** est cylindrique, aimanté diamétralement et collé, par exemple à l'aide d'une colle silicone, à l'intérieur de deux premier **31** et second **32** capuchons tête-bêches qui, à l'extrémité opposée à celle dans laquelle est inséré ledit aimant permanent **3,** sont respectivement prolongés, par un premier axe **311** qui matérialise l'axe de rotation dudit aimant permanent **3** puis par un axe excentrique **52,** pour ledit premier capuchon **31,** et par un second axe **321** qui matérialise l'axe de rotation dudit aimant permanent **3** seulement, pour ledit second capuchon **32.** En outre, un levier **51** solidaire d'un arbre d'entrée **41** dont il partage l'axe de rotation, comporte une gorge d'appui **511** dont la largeur est supérieure ou égale, mais préférentiellement égale dans ce second mode de réalisation préféré, au diamètre dudit axe excentrique **52** avec lequel elle coopère pour constituer lesdits moyens démultiplicateurs.

L'arbre de sortie **42** comporte deux surfaces cylindriques destinées, l'une **422** à recevoir ledit ressort de roue libre d'entraînement **43,** l'autre **423** à recevoir ledit ressort de maintien **44** et, accolé à la première, un axe **421** qui constitue l'axe de rotation dudit arbre d'entrée **41** et, accolée à la seconde, une vis **61** qui coopère avec un taraudage **62** parallèle à l'axe de ladite seconde partie **2** pour constituer lesdits moyens de transmission pour que la rotation dudit arbre de sortie **42** entraîne le déplacement de ladite seconde partie **2** par rapport à ladite première partie **1.**

Un support **36** dont la surface extérieure est essentiellement cylindrique et apte à coopérer avec ladite cavité cylindrique **15,** à l'exception d'une protubérance **364** qui est apte à coopérer avec ladite gorge intérieure longitudinale **151** dudit tube **10** pour assurer le blocage en rotation dudit support **36** par rapport audit tube **10,** reçoit, en les maintenant dans une disposition relative adaptée :
- ledit premier axe **311** auquel il sert de palier dans un premier perçage **361** axial,
- l'axe **421** dudit arbre de sortie **42** dans un second perçage **362** parallèle audit premier perçage **361,** ledit arbre d'entrée **41** ayant été préalablement enfilé sur cet axe **421,**
- le levier **51** dans un troisième perçage **363** perpendiculaire aux dits premier **361** et second **362** perçages, la surface dudit troisième perçage **363** limitant la rotation dudit levier **51** dont les extrémités **512** et **513** viennent respectivement en butée sur ledit troisième perçage **363** quand le levier **51** tourne dans ledit premier sens ou tourne dans le sens opposé audit premier sens. L'ensemble constituant des premiers moyens de butée dans un sens et des seconds moyens de butée dans le sens opposé pour limiter la rotation dudit aimant permanent 3 à un secteur d'angle inférieur à 180°, si les dimensions dudit levier **51** et dudit troisième perçage **363** sont convenablement choisies.

Le rapport de réduction de ce second mode préféré de réalisation du dispositif suivant l'invention est fonction de la distance entre ledit premier perçage **361** et ledit second perçage **362,** d'une part, et de l'excentration dudit axe excentrique **52,** d'autre part.

Un bouchon **35,** muni d'un perçage axial **351** qui reçoit ledit second axe **321** auquel il sert de palier, s'emboîte en force sur ladite première extrémité dudit tube **10** par l'intermédiaire d'une surface cylindrique **352** de diamètre légèrement supérieur à celui de ladite cavité cylindrique **15.**

Une pièce d'appui **37** qui prend place au fond de ladite cavité cylindrique **15** en butée sur ladite surface annulaire d'appui **152** et est bloquée en rotation par le contact de sa surface plane longitudinale **373** avec une autre surface plane longitudinale **365** dudit support **36,** porte également une surface cylindrique de friction **371** apte à coopérer avec le ressort de maintien **44** pour constituer lesdits moyens de maintien pour empêcher la rotation dudit arbre de sortie **42** dans le sens opposé audit premier sens. Cette pièce d'appui **37** supporte également tout le poids appliqué par le patient sur le clou d'allongement et transféré par l'intermédiaire de ladite seconde partie **2,** de ladite vis **61,** qui travaille en traction, et de la partie dudit arbre de sortie **42** qui est au contact de ladite pièce d'appui **37.**

Le fonctionnement de ce second mode de réalisation préféré du dispositif suivant l'invention est très similaire à celui dudit premier mode de réalisation préféré du dispositif suivant l'invention et ne sera donc pas explicité dans le détail. Les figures 9 et 10 montrent le déplacement de ladite seconde partie **2** par rapport à ladite première partie **1** entre le moment où le clou d'allongement est implanté, figure 9, et la fin de l'allongement, figure 10. Les figures 11 et 12 montrent les positions en butée dudit levier **51** et les directions d'aimantation dudit aimant permanent **3** obtenues sous l'effet d'une source de champ magnétique **81** placée à l'extérieur de l'organisme. Sur ces figures, les flèches courbes indiquent les rotations déjà effectuées de la direction d'aimantation dudit aimant permanent **3** et dudit levier **51** pour arriver à la position de butée représentée et non pas le mouvement qui va s'accomplir. Ces flèches sont sensiblement à l'échelle des rotations accomplies et témoignent donc du rapport de réduction du dispositif suivant l'invention.

Lesdites figures représentent des états stables et il faut modifier la position de ladite source de champ magnétique **81,** par exemple la retourner, pour qu'ils évoluent vers l'état représenté sur l'autre figure.

Le troisième mode de réalisation préféré du dispositif suivant l'invention représenté sur les figures 13 et 14 et auxquelles on se réfère maintenant est particulièrement intéressant quand on souhaite obtenir un rapport de réduction très élevé dans un diamètre particulièrement faible quand on dispose, en revanche, de suffisamment de longueur. En contrepartie, la commande de ce troisième mode de réalisation préféré peut devenir fastidieuse si elle doit être faite manuellement.

La première partie **1** dudit troisième mode de réalisation préféré du dispositif suivant l'invention est sensiblement un cylindre avec une extrémité arrondie qui porte également au moins un perçage sensiblement perpendiculaire à l'axe de ladite première partie 1 et qui constitue lesdits premier moyens **11** de liaison à l'organisme. A l'extrémité opposée à ladite extrémité arrondie se trouvent, disposées radialement à la surface dudit cylindre, des ailettes anti-rotation **17,** et, dans le prolongement dudit cylindre et partageant son axe, une surface cylindrique **141** apte à coopérer avec un ressort de **44** et un logement cylindrique **18** qui débouche à l'extrémité de ladite surface cylindrique **141** opposée à celle qui la rattache audit cylindre.

La seconde partie **2** dudit troisième mode de réalisation préféré du dispositif suivant l'invention est un tube entièrement fileté sur sa face interne **62** dans lequel ladite première partie **1** peut se translater librement mais sans jeu excessif. Des gorges anti-rotation **22** aptes à coopérer avec lesdites ailettes anti-rotation **17** de ladite première partie 1 de manière à empêcher la rotation de l'une par rapport à l'autre, sont découpées longitudinalement à l'intérieur de ladite seconde partie **2.** Sensiblement à une extrémité de ladite seconde partie **2**, au moins un perçage faisant un angle de 30 à 90° avec l'axe constitue lesdits second moyens **21** de liaison à l'organisme.

L'aimant permanent **3** est cylindrique, aimanté diamétralement et collé, par exemple à l'aide d'une colle silicone, à l'intérieur d'un capuchon **37** qui, à l'extrémité opposée à celle dans laquelle est introduite ledit aimant permanent **3** comporte un taraudage **371** qui reçoit une vis **38** comportant une partie filetée **53,** ou quatrième surface, et une tête **381** et qui est soudée en bout du côté opposé à sa tête **381** à l'intérieur dudit capuchon **37** avant collage dudit aimant permanent **3.** Ladite vis **38** matérialise l'axe de rotation dudit aimant permanent **3** dont elle est totalement solidaire. La tête **381** de ladite vis **38** est logée dans ledit logement cylindrique **18** de ladite première partie **1.** Son corps est introduit dans le canal central **393** débouchant de part et d'autre d'un axe d'assemblage **39** qui comporte un corps cylindrique **391** dont le diamètre est légèrement supérieur à celui dudit logement cylindrique **18** et une tête **392** et sur lequel a été préalablement monté dans l'ordre ledit arbre d'entrée **41** contre la tête **392** puis, ledit ressort de roue libre d'entraînement **43,** ledit arbre de sortie **42** et ledit ressort de maintien **44.** L'extrémité dudit corps cylindrique **391** opposée à ladite tête 392 est assemblée en force dans ledit logement cylindrique **18** bloquant en translation mais pas en rotation ladite vis **38** et donc ledit aimant permanent **3** par rapport à ladite première partie **1** et maintenant accolés les uns aux autres les éléments qui ont été préalablement montés sur ledit corps cylindrique **391.**

Ledit arbre de sortie **42** comporte deux surfaces cylindrique destinées, l'une **422** à recevoir ledit ressort de roue libre d'entraînement **43,** l'autre **423** à recevoir ledit ressort de maintien **44** et disposées de part et d'autre d'une vis **61** apte à coopérer avec l'intérieur taraudé **62** de ladite seconde partie **2 .** Le centre dudit arbre de sortie **42** comporte un perçage **424** qui permet audit arbre de sortie **42** d'être monté libre en rotation sur ledit axe d'assemblage **39.** Tous ces éléments sont coaxiaux.

Ledit arbre d'entrée **41** comporte, à une extrémité une surface cylindrique destinée à recevoir ledit ressort de roue libre d'entraînement **43** et, à l'autre extrémité, une torsade **55,** ou sixième surface. Il est également traversé suivant son axe par un perçage **411** qui lui permet d'être monté libre en rotation sur ledit axe d'assemblage **39.**

Ce troisième mode de réalisation préféré du dispositif suivant l'invention comporte en outre une pièce amplificatrice **54** sensiblement cylindrique et apte à coulisser dans ladite seconde partie **2** et qui comprend des ailettes anti-rotation **543** aptes à coopérer avec lesdites gorges anti-rotation **22** de ladite seconde partie **2,** qui est elle-même bloquée en rotation avec ladite première partie, et, à une extrémité, un taraudage **541,** ou troisième surface, apte à coopérer avec la partie filetée **53** de ladite vis **38** et, à l'autre extrémité, des surfaces femelles **542,** ou cinquième surface, aptes à coopérer avec ladite torsade **55.** Cette pièce amplificatrice **54** vient se visser sur ladite partie filetée **53** de ladite vis **38** après assemblage en force dudit axe d'assemblage **39** dans ladite première partie **1** et avant vissage dudit capuchon **37** sur ladite vis **38** et soudure en bout de l'un avec l'autre.

Ce troisième mode de réalisation préféré du dispositif suivant l'invention fonctionne de la manière suivante: un champ magnétique tournant créé par la source de champ magnétique placée à l'extérieur de l'organisme met ledit aimant permanent **3** en rotation dans un premier sens pour un nombre de tours dont le maximum est imposé par la longueur et le pas de la partie filetée **53** de la vis **38** ou par l'espace réservé à la pièce amplificatrice **54** pour sa translation entre les butées constituées par le capuchon **37** d'une part et la tête **392** dudit axe d'assemblage **39** d'autre part. La rotation dudit aimant permanent **3** entraîne celle de ladite vis **38** dont il est solidaire et, par l'intermédiaire de la partie filetée **53** de cette dernière, qui coopèrent avec le taraudage **541** de ladite pièce amplificatrice **54** pour constituer ladite seconde liaison hélicoïdale, la translation de ladite pièce amplificatrice **54** dans une premier sens de translation. La pièce amplificatrice étant bloquée en rotation par les ailettes anti-rotation **543** et coopérant par l'intermédiaire de ses surfaces femelles **542** avec ladite torsade **55** entraîne ledit arbre d'entrée **41** en rotation dans un sens qui dépend bien sûr aussi du sens du pas de ladite torsade **55.**

Puis, ledit aimant permanent **3** est mis rotation dans le sens opposé audit premier sens pour un nombre de tours identique à celui effectué dans ledit premier sens, et ledit arbre d'entrée **41** retrouve sa position initiale par l'effet du mouvement inverse de toutes les pièces. Pendant ces mouvements, le système de ressorts de frictions fait son travail et entraîne ledit arbre de sortie **42** en rotation dans un seul sens ce qui conduit de manière évidente au déplacement de ladite seconde partie **2** par rapport à ladite première partie **1.** On réitère ce cycle autant de fois que nécessaire pour obtenir l'allongement souhaité.

Le rapport de réduction de ce troisième mode préféré de réalisation du dispositif suivant l'invention est fonction des pas de ladite partie filetée 53 de ladite vis et de ladite torsade **55.** En outre, le pas de ladite torsade **55** doit être suffisamment grand au regard des frottements existants pour que ledit dispositif fonctionne.

Dans ce troisième mode de réalisation préféré du dispositif suivant l'invention, il est aussi possible d'intercaler un mécanisme à croix de Malte entre l'aimant permanent **3** et la vis **53** pour faciliter, si elle est souhaitable, la commande dudit dispositif par rotation d'une source de champ magnétique **81** externe à l'organisme autour d'un axe parallèle à l'axe de rotation dudit aimant permanent **3** et placé au plus près dudit aimant permanent **3.** En effet l'utilisation d'une croix de Malte permet que ledit aimant permanent **3** n'entraîne l'arbre d'entrée **41** que sur une portion de sa rotation pour laquelle le couple qui peut être exercé sur ledit aimant permanent **3** par ladite source de champ magnétique **81** est élevé grâce à un calage judicieux de la direction d'aimantation dudit aimant permanent **3** par rapport à la direction dans laquelle on place la source de champ magnétique **81** et à la position de la roue, solidaire dudit aimant permanent **3**, qui entraîne la croix de Malte, solidaire de ladite vis **53,** par rapport à ladite croix de Malte. Un bon résultat est obtenu si la bissectrice de la direction d'aimantation dudit aimant permanent **3** lorsque ladite roue d'entraînement commence à engrener ladite croix de Malte et la direction d'aimantation dudit aimant permanent **3** lorsque ladite roue d'entraînement quitte ladite croix de Malte est sensiblement perpendiculaire à la direction dans laquelle on peut placer ladite source de champ magnétique **81** au plus près dudit aimant permanent **3.**

Le dispositif suivant l'invention est avantageusement réalisé dans des matériaux résistants mécaniquement et bien tolérés par l'organisme tels des aciers inoxydables comme le 316L, des alliages de titane ou, de préférence, comme des alliages hautes performances à base de chrome et de cobalt comme par exemple le PHYNOX. Ledit aimant permanent **3** est avantageusement un aimant néodyme du type de ceux dont la température de Curie est supérieure à 150 °C pour permettre la stérilisation dudit dispositif suivant l'invention par tous moyens et notamment à la vapeur surchauffée à 134°C sans risque de détérioration dudit aimant permanent **3.**

Avantageusement les surfaces soumises à un frottement dudit dispositif suivant l'invention reçoivent un traitement de surface diminuant leur coefficient de frottement à base de carbone amorphe diamantin ou de de bisulfure de tungstène par exemple.

L'utilisation d'un aimant permanent **3** cylindrique aimanté diamétralement a été décrite et est préférée mais toute géométrie d'aimant est envisageable en particulier parallélépipédique, à section hexagonale etc... avec toute direction d'aimantation.

Le dispositif suivant l'invention s'implante et se fixe à l'intérieur de l'organisme de manière tout à fait connue et similaire à ses homologues non dotés d'un mécanisme d'allongement.

### Possibilités d'application industrielle

La présente invention est particulièrement utile pour la réalisation de tout type de dispositif d'allongement intra-corporel tels que des prothèses osseuses à croissances, des clous médullaires d'allongement ou de transport osseux, des tiges rachidiennes de distraction ou de compression ou des distracteurs osseux ou inter-costaux. Elle peut aussi servir à la réalisation de plaques à croissance pour la correction de déformations osseuses ou de pompes implantées pour la diffusion progressive et contrôlée de substances dans l'organisme et, plus largement, peut permettre la réalisation de tout type de dispositif dont on souhaite pouvoir modifier la géométrie de manière graduelle et contrôlée sans contact direct.

## Revendications

1. Dispositif d'allongement intra-corporel constitué d'une première partie **(1)** qui comprend des premiers moyens **(11)** de liaison à l'organisme, d'une seconde partie **(2)** qui comprend des seconds moyens **(21)** de liaison à l'organisme et montée coulissante par rapport à la première partie **(1)** et d'un aimant permanent **(3),** le dispositif comportant en outre:
- des moyens de liaison entre l'aimant permanent **(3)** et la première partie **(1)** laissant à l'aimant permanent **(3)** un degré de liberté en rotation sensiblement autour d'un de ses axes principaux d'inertie perpendiculaire à sa direction d'aimantation,
- un arbre d'entrée **(41),**
- un arbre de sortie (42),
- des moyens de transmission pour que la rotation de l'arbre de sortie (42) entraîne le déplacement de la seconde partie (2) par rapport à la première partie (1),
le dispositif étant **caractérisé en ce qu'**il comporte:
- des moyens démultiplicateurs transformant les mouvements de l'aimant permanent **(3)** en rotation de l'arbre d'entrée **(41)** alternativement dans un premier sens et dans le sens opposé avec un rapport de réduction entre la rotation de l'arbre d'entrée **(41)** et celle de l'aimant permanent **(3),**
- des moyens de roue libre **(43)** aptes à entraîner l'arbre de sortie **(42)** dans le premier sens quand l'arbre d'entrée **(41)** tourne dans le premier sens,
- des moyens de maintien **(44)** pour empêcher la rotation de l'arbre de sortie **(42)** dans le sens opposé au premier sens quand l'arbre d'entrée **(41)** tourne dans le sens opposé au premier sens.

2. Dispositif d'allongement intra-corporel selon la revendication 1, **caractérisé en ce que** les moyens de transmission sont une première liaison hélicoïdale entre une première surface solidaire **(61)** de l'arbre de sortie **(42)** et une seconde surface **(62)** solidaire de la seconde partie **(2).**

3. Dispositif d'allongement intra-corporel selon la revendication 1, **caractérisé en ce que** les moyens démultiplicateurs sont constitués d'un levier **(51)** solidaire de l'arbre d'entrée **(41)** dont il partage l'axe de rotation et de moyens de liaison entre une première surface d'appui **(52)** solidaire de l'aimant permanent **(3)** et distante de son axe de rotation et une seconde surface d'appui **(511)** solidaire de l'extrémité du levier **(51)** la plus éloignée de l'axe de rotation commun au levier **(51)** et à l'arbre d'entrée **(41).**

4. Dispositif d'allongement intra-corporel selon la revendication 3, **caractérisé en ce que** la rotation de l'aimant permanent **(3)** est limitée à un secteur d'angle inférieur à 180° par des premiers moyens de butée **(512)** dans un sens et des seconds moyens de butée **(513)** dans le sens opposé.

5. Dispositif d'allongement intra-corporel selon l'une quelconque des revendications 3 ou 4, **caractérisé en ce que** les moyens de liaison entre la première surface d'appui **(52)** et la seconde surface d'appui **(511)** comportent un jeu qui permet la rotation de l'aimant permanent **(3)** sans entraînement du levier **(51)** sur deux secteurs sensiblement opposés l'un à l'autre par rapport à l'axe de rotation de l'aimant permanent **(3).**

6. Dispositif d'allongement intra-corporel constitué d'une première partie **(1)** qui comprend des premiers moyens **(11)** de liaison à l'organisme, d'une seconde partie **(2)** qui comprend des seconds moyens **(21)** de liaison à l'organisme et montée coulissante par rapport à la première partie **(1)** et d'un aimant permanent **(3),** le dispositif comportant en outre:
- des moyens de liaison entre l'aimant permanent (3) et la première partie **(1)** laissant à l'aimant permanent **(3)** un degré de liberté en rotation sensiblement autour d'un de ses axes principaux d'inertie perpendiculaire à sa direction d'aimantation,
- un arbre d'entrée **(41),**
- des moyens démultiplicateurs transformant les mouvements de l'aimant permanent **(3)** en rotation de l'arbre d'entrée **(41)** alternativement dans un premier sens ou dans le sens opposé avec un rapport de réduction entre la rotation de l'arbre d'entrée **(41)** et celle de l'aimant permanent **(3)** et tels qu'un changement de sens de la rotation de l'aimant permanent **(3)** entraîne le changement de sens de la rotation de l'arbre d'entrée **(41),**
- un arbre de sortie **(42),**
- des moyens de transmission pour que la rotation de l'arbre de sortie **(42)** entraîne le déplacement de la seconde partie (2) par rapport à la première partie (1),
le dispositif étant caractérisé en qu'il comporte:
- des moyens de roue libre (43) aptes à entraîner l'arbre de sortie (42) dans le premier sens quand l'arbre d'entrée (41) tourne dans le premier sens,
- des moyens de maintien (44) pour empêcher la rotation de l'arbre de sortie (42) dans le sens opposé au premier sens quand l'arbre d'entrée (41) tourne dans le sens opposé au premier sens.

7. Dispositif d'allongement intra-corporel selon la revendication 6, **caractérisé en ce que** les moyens démultiplicateurs sont constitués d'une pièce amplificatrice **(54)** de l'arbre d'entrée **(41)** dont il partage l'axe de rotation et de moyens de liaison entre une surface femelle **(542)** solidaire de l'aimant permanent **(3)** et une torsade **(55)** solidaire de la pièce amplificatrice **(54).**

8. Dispositif d'allongement intra-corporel selon la revendication 6, **caractérisé en ce que** les moyens démultiplicateurs sont constitués d'une pièce amplificatrice **(54)** montée en translation et bloquée en rotation par rapport à la première partie **(1)** et comportant une troisième surface **(541)** en seconde liaison hélicoïdale avec une quatrième surface **(53)** solidaire de l'aimant permanent, et une cinquième surface **(542)** en troisième liaison hélicoïdale avec une sixième surface **(55)** solidaire de l'arbre d'entrée **(41),** les axes de rotation de l'aimant permanent **(3),** des seconde et troisième liaisons hélicoïdale et de l'arbre d'entrée **(41)** étant confondus, les pas des seconde et troisième liaisons hélicoïdales étant dans le rapport de réduction des moyens démultiplicateurs et le pas de la seconde liaison hélicoïdale étant suffisamment grand relativement au coefficient de friction de la seconde liaison hélicoïdale pour permettre le fonctionnement des moyens démultiplicateurs.

## Patentansprüche

1. Intrakorporale Verlängerungsvorrichtung, die aus einem ersten Teil (1), der erste Mittel (11) zur Verbindung mit dem Organismus aufweist, aus einem zweiten Teil (2), der zweite Mittel (21) zur Verbindung mit dem Organismus aufweist und gleitbar gegenüber dem ersten Teil (1) befestigt ist, und einem Permanentmagneten (3) gebildet ist, wobei die Vorrichtung ferner Folgendes aufweist:
- Verbindungsmittel zwischen dem Permanentmagneten (3) und dem ersten Teil (1), die dem Permanentmagneten (3) einen Freiheitsgrad der Drehung im Wesentlichen um eine seiner Hauptträgheitsachsen senkrecht zu seiner Magnetisierungsrichtung lassen,
- eine Eingangswelle (41),
- eine Ausgangswelle (42),
- Übertragungsmittel, damit die Drehung der Ausgangswelle (42) die Verschiebung des zweiten Teils (2) bezüglich des ersten Teils (1) antreibt,
wobei die Vorrichtung **dadurch gekennzeichnet ist, dass** sie Folgendes aufweist:
- Untersetzungsmittel, die die Bewegungen des Permanentmagneten (3) in Drehung der Eingangswelle (41) abwechselnd in eine erste Richtung und die entgegengesetzte Richtung mit einem Untersetzungsverhältnis zwischen der Drehung der Eingangswelle (41) und jener des Permanentmagneten (3) verwandeln,
- Freilaufmittel (43), die geeignet sind, die Ausgangswelle (42) in die erste Richtung anzutreiben, wenn sich die Eingangswelle (41) in der ersten Richtung dreht,
- Haltemittel (44), um die Drehung der Ausgangswelle (42) in der entgegengesetzten Richtung zu der ersten Richtung zu verhindern, wenn sich die Eingangswelle (41) in der entgegengesetzten Richtung zu der ersten Richtung dreht.

2. Intrakorporale Verlängerungsvorrichtung nach Anspruch 1, **dadurch gekennzeichnet, dass** die Übertragungsmittel eine erste spiralförmige Verbindung zwischen einer ersten Fläche (61), die mit der Ausgangswelle (42) fest verbunden ist, und einer zweiten Fläche (62), die mit dem zweiten Teil (2) fest verbunden ist, sind.

3. Intrakorporale Verlängerungsvorrichtung nach Anspruch 1, **dadurch gekennzeichnet, dass** die Untersetzungsmittel aus einem Hebel (51), der mit der Eingangswelle (41) fest verbunden ist, mit der er die Drehachse teilt, und aus Verbindungsmitteln zwischen einer ersten Auflagefläche (52) gebildet sind, die mit dem Permanentmagneten (3) fest verbunden ist und von seiner Drehachse entfernt ist, und einer zweiten Auflagefläche (511), die mit dem Ende des Hebels (51) fest verbunden ist, das von der Drehachse am weitesten entfernt ist, die dem Hebel (51) und der Eingangswelle (41) gemeinsam ist.

4. Intrakorporale Verlängerungsvorrichtung nach Anspruch 3, **dadurch gekennzeichnet, dass** die Drehung des Permanentmagneten (3) auf einen Winkelsektor, der niedriger als 180 ° ist, durch erste Anschlagwinkel (512) in eine Richtung und zweite Anschlagmittel (513) in die entgegengesetzte Richtung begrenzt ist.

5. Intrakorporale Verlängerungsvorrichtung nach einem der Ansprüche 3 oder 4, **dadurch gekennzeichnet, dass** die Verbindungsmittel zwischen der ersten Auflagefläche (52) und der zweiten Auflagefläche (511) ein Spiel aufweisen, das die Drehung des Permanentmagneten (3) ohne Antrieb des Hebels (51) auf zwei im Wesentlichen zueinander entgegengesetzten Sektoren gegenüber der Drehachse des Permanentmagneten (3) ermöglicht.

6. Intrakorporale Verlängerungsvorrichtung, die aus einem ersten Teil (1), der erste Mittel (11) zur Verbindung mit dem Organismus aufweist, aus einem zweiten Teil (2), der zweite Mittel (21) zur Verbindung mit dem Organismus aufweist und gleitbar gegenüber dem ersten Teil (1) befestigt ist, und einem Permanentmagneten (3) gebildet ist, wobei die Vorrichtung ferner Folgendes aufweist:
- Verbindungsmittel zwischen dem Permanentmagneten (3) und dem ersten Teil (1), die dem Permanentmagneten (3) einen Freiheitsgrad der Drehung im Wesentlichen um eine seiner Hauptträgheitsachsen senkrecht zu seiner Magnetisierungsrichtung lassen,
- eine Eingangswelle (41),
- Untersetzungsmittel, die die Bewegungen des Permanentmagneten (3) in Drehung der Eingangswelle (41) abwechselnd in eine erste Richtung oder in die entgegengesetzte Richtung mit einem Untersetzungsverhältnis zwischen der Drehung der Eingangswelle (41) und jener des Permanentmagneten (3) verwandeln und derart sind, dass eine Richtungsänderung der Drehung des Permanentmagneten (3) die Richtungsänderung der Drehung der Eingangswelle (41) bewirkt,
- eine Ausgangswelle (42),
- Übertragungsmittel, damit die Drehung der Ausgangswelle (42) die Verschiebung des zweiten Teils (2) bezüglich des ersten Teils (1) antreibt,
wobei die Vorrichtung **dadurch gekennzeichnet ist, dass** sie Folgendes aufweist:
- Freilaufmittel (43), die geeignet sind, die Ausgangswelle (42) in die erste Richtung anzutreiben, wenn sich die Eingangswelle (41) in der ersten Richtung dreht,
- Haltemittel (44), um die Drehung der Ausgangswelle (42) in der entgegengesetzten Richtung zu der ersten Richtung zu verhindern, wenn sich die Eingangswelle (41) in der entgegengesetzten Richtung zu der ersten Richtung dreht.

7. Intrakorporale Verlängerungsvorrichtung nach Anspruch 6, **dadurch gekennzeichnet, dass** die Untersetzungsmittel aus einem Verstärkungsteil (54) der Eingangswelle (41), mit der es die Drehachse teilt, und aus Verbindungsmitteln zwischen einer aufnehmenden Fläche (542), die mit dem Permanentmagneten (3) fest verbunden ist, und einer Kordel (55), die mit dem Verstärkungsteil (54) fest verbunden ist, gebildet sind.

8. Intrakorporale Verlängerungsvorrichtung nach Anspruch 6, **dadurch gekennzeichnet, dass** die Untersetzungsmittel aus einem Verstärkungsteil (54) gebildet sind, das gegenüber dem ersten Teil (1) translatorisch befestigt und in Drehung blockiert ist und eine dritte Fläche (541), die in zweiter spiralförmiger Verbindung mit einer vierten Fläche (53) ist, die mit dem Permanentmagneten fest verbunden ist, und eine fünfte Fläche (542), die in dritter spiralförmiger Verbindung mit einer sechsten Fläche (55) ist, die mit der Eingangswelle (41) fest verbunden ist, aufweist, wobei die Drehachsen des Permanentmagneten (3) der zweiten und dritten spiralförmigen Verbindung der Eingangswelle (41) zusammenfallen, wobei die Gewinde der zweiten und dritten spiralförmigen Verbindung in dem Übersetzungsverhältnis der Untersetzungsmittel sind und das Gewinde der zweiten spiralförmigen Verbindung ausreichend groß relativ zu dem Reibungskoeffizienten der zweiten spiralförmigen Verbindung ist, um das Funktionieren der Untersetzungsmittel zu ermöglichen.

## Claims

1. Intracorporeal elongation device composed of a first part (1), which comprises first means (11) of connection to the body, a second part (2), which comprises second means (21) of connection to the body and is mounted so as to slide relative to the first part (1), and a permanent magnet (3), the device further including:
- means of connection between the permanent magnet (3) and the first part (1) allowing the permanent magnet (3) a degree of freedom of rotation substantially about one of its main axes of inertia perpendicular to its direction of magnetization,
- an input shaft (41),
- an output shaft (42),
- transmission means such that the rotation of the output shaft (42) causes the displacement of the second part (2) relative to the first part (1),
the device being **characterized in that** it includes:
- step-down gears transforming the movements of the permanent magnet (3) into rotation of the input shaft (41), alternately in a first direction and in the opposite direction, with a gear reduction ratio between the rotation of the input shaft (41) and that of the permanent magnet (3),
- free-wheel means (43) for driving the output shaft (42) in the first direction when the input shaft (41) turns in the first direction,
- retaining means (44) for preventing the rotation of the output shaft (42) in the direction opposite to the first direction when the input shaft (41) turns in the direction opposite to the first direction.

2. Intracorporeal elongation device according to Claim 1, **characterized in that** the transmission means are a first helical connection between a first surface (61) integral with the output shaft (42) and a second surface (62) integral with the second part (2).

3. Intracorporeal elongation device according to Claim 1, **characterized in that** the step-down gears are formed by a lever (51) integral with the input shaft (41), with which it shares the axis of rotation, and means of connection between a first supporting surface (52), integral with the permanent magnet (3) and remote from its axis of rotation, and a second supporting surface (511) integral with the end of the lever (51) farthest from the axis of rotation common to the lever (51) and to the input shaft (41).

4. Intracorporeal elongation device according to Claim 3, **characterized in that** the rotation of the permanent magnet (3) is limited to an angle sector of less than 180° by first abutment means (512) in one direction, and by second abutment means (513) in the opposite direction.

5. Intracorporeal elongation device according to either of Claims 3 and 4, **characterized in that** the means of connection between the first supporting surface (52) and the second supporting surface (511) include a clearance that permits the rotation of the permanent magnet (3), without driving the lever (51), on two sectors substantially opposite to each other relative to the axis of rotation of the permanent magnet (3).

6. Intracorporeal elongation device composed of a first part (1), which comprises first means (11) of connection to the body, a second part (2), which comprises second means (21) of connection to the body and is mounted so as to slide relative to the first part (1), and a permanent magnet (3), the device further including:
- means of connection between the permanent magnet (3) and the first part (1) allowing the permanent magnet (3) a degree of freedom of rotation substantially about one of its main axes of inertia perpendicular to its direction of magnetization,
- an input shaft (41),
- step-down gears transforming the movements of the permanent magnet (3) into rotation of the input shaft (41), alternately in a first direction or in the opposite direction, with a gear reduction ratio between the rotation of the input shaft (41) and that of the permanent magnet (3) and such that a change of direction of rotation of the permanent magnet (3) causes the change of direction of rotation of the input shaft (41),
- an output shaft (42),
- transmission means such that the rotation of the output shaft (42) causes the displacement of the second part (2) relative to the first part (1),
The device being **characterized in that** it includes:
- free-wheel means (43) for driving the output shaft (42) in the first direction when the input shaft (41) turns in the first direction,
- retaining means (44) for preventing the rotation of the output shaft (42) in the direction opposite to the first direction when the input shaft (41) turns in the direction opposite to the first direction.

7. Intracorporeal elongation device according to Claim 6, **characterized in that** the step-down gears are formed by an amplifying component (54) of the input shaft (41), with which it shares the axis of rotation, and means of connection between a female surface (542), integral with the permanent magnet (3), and a torsade (55) integral with the amplifying component (54).

8. Intracorporeal elongation device according to Claim 6, **characterized in that** the step-down gears are formed by an amplifying component (54) mounted in translation and blocked in rotation relative to the first part (1) and comprising a third surface (541) in second helical connection to a fourth surface (53) integral with the permanent magnet, and a fifth surface (542) in third helical connection to a sixth surface (55) integral with the input shaft (41), the axes of rotation of the permanent magnet (3), of the second and third helical connections and of the input shaft (41) being coincident, the pitches of the second and third helical connections being in the gear reduction ratio of the step-down gears, and the pitch of the second helical connection being sufficiently great relative to the coefficient of friction of the second helical connection to permit the functioning of the step-down gears.
